# EUROPEAN PATENT SPECIFICATION

(11) **EP 1 307 205 B1**
(45) Date of publication and mention of the grant of the patent: **04.04.2007**
(21) Application number: 01905886.6
(22) Date of filing: 15.02.2001
(51) Int. Cl.: A61K 31/565, A61K 31/567, A61K 31/5685, A61K 31/57, A61P 9/10, A61P 25/28

(54) **USE OF 7ALPHA-HYDROXY-ESTRADIOL, 7ALPHA-HYDROXY-DEHYDROEPIANDROSTERONE AND 7ALPHA-HYDROXY-PREGNENOLONE DERIVATIVES FOR TREATING ACUTE CELLULAR DEGENERATION**
VERWENDUNG VON 7ALPHA-HYDROXY-ESTRADIOL, 7ALPHA-HYDROXY-DEHYDROEPIANDROSTERON AND 7ALPHA-HYDROXY-PREGNENOLON DERIVATEN ZUR BEHANDLUNG DER AKUTEN ZELLULÄREN DEGENERATION
UTILISATION DE DERIVES DU 7ALPHA-HYDROXY-OESTRADIOL, 7ALPHA-HYDROXY-DEHYDROEPIANDROSTERONE ET DE LA 7ALPHA-HYDROXY-PREGNENOLONE POUR TRAITER LA DEGENERATION CELLULAIRE AIGUE

(30) Priority: 15.02.2000 GB 0003524
(43) Date of publication of application: 07.05.2003
(73) Proprietor: THE UNIVERSITY OF EDINBURGH, South Bridge, Edinburgh EH8 9YL (GB)
(72) Inventor: LATHE, Richard Frank, Edinburgh EH9 3JQ (GB); SECKL, Jonathan Robert, University of Edinburgh, Edinburgh EH4 2XU (GB); MARTIN, Keith Frank, Limeburner Lane, London EC4M 7SB (GB); WULFERT, Ernst, Arne, 1180 Brussels (BE)
(74) Representative: Chapman, Paul Gilmour
(86) International application number: PCT/GB2001/000627
(87) International publication number: WO 2001/060375

(56) References cited:
- WO-A-94/03176
- WO-A-94/08588
- WO-A-95/12402
- WO-A-96/40152
- WO-A-97/03677
- WO-A-97/37664
- WO-A-98/40074
- GB-A- 1 528 796
- PARK-CHUNG, MIJEONG ET AL: "Distinct sites for inverse modulation of N-methyl-D-aspartate receptors by sulfated steroids" MOL. PHARMACOL. (1997), 52(6), 1113-1123, XP000911767
- IRWIN, ROBERT P. ET AL: "Steroid potentiation and inhibition of N-methyl-D-aspartate receptor-mediated intracellular Ca++ responses: structure-activity studies" J. PHARMACOL. EXP. THER. (1994), 271(2), 677-82, XP000911758
- Y. AKWA ET AL.: "Astrocytes and Neurosteroids: Metabolism of Pregnenolone and Dehydroepiandrosterone. Regulation by Cell Density" THE JOURNAL OF CELL BIOLOGY, vol. 121, no. 1, 1993, pages 135-143, XP000911759

## Description

The present invention relates to the use of known and novel steroids in the treatment of conditions that lead to damage and death of cells, particularly of nerve cells and more particularly of CNS nerve cells. The use particularly relates to treatment of acute conditions affecting the CNS, such as stroke and head or spinal trauma, wherein treatment within days, or even hours, of onset of the condition is required to prevent irreversible damage and/or death.

The 7α-hydroxylated derivatives of estradiol, ie. 3β-, 7α-, 17β-estradiol, and their 7-keto precursors have been known for many years. US 2,418,603 describes the use of 7α-hydroxy and/or 7-keto-estradiol in the synthesis of estradiol and ascribes to them at most 1/300 of the estrogenic activity of estrone. The 7α-hydroxy compounds at least are known to be a naturally occurring metabolites of estrogens and related compounds (see Holler et al (1984) J. Steroid Biochem Vol 20, No 3 pp785-787).

More recently 7α-hydroxy-estradiols and -triols have been proposed for use as medicaments for post-menopausal patients due to their vaginotropic activity (see BE 834,489, GB 1,528,796), these preferably being ethynyl substituted at the 17 position. 7α-hydroxy-estradiol itself (herein 7OHE2) is reported to have anti-implantation activity while having low estrogenic activity (see eg. Muller and Wotiz, (1977) Endo, Vol 100, No 2; Wiese et al (1997) 40, p3659-3669) and to have low glutamate dehydrogenase inhibiting activity compared to most estradiols (Pons et al (1978) 84, p257-266). The corresponding 17-desoxy estrogens appear to have similarly low estrogenic activity (Peters et al (1989) J.Am. Chem. 32. p1632-1652).

In the last 10 years the potential role of 7α-hydroxy and 7-keto DHEA and pregnenolone as antiglucocorticoids has been highlighted by several groups (see for example US 5,707,983, US 5,763,433 and US 5,976,850) and the DHEA compounds have been suggested for use in treatment of Alzheimer's disease due to their theoretical sharing of postulated benefits of DHEA coupled to lack of sex hormone like characteristics.

It has further been determined that transcripts of the enzyme CYP7B are reduced in Alzheimer's disease and that this enzyme has activity in converting DHEA, pregnenolone and estradiol to their corresponding 7α-hydroxy derivatives. Relative activity of the enzyme against substrates is in the order DHEA>pregnenolone>estradiol, with the implication being that greater effect of enzyme depletion would be seen on the antiglucocorticoids 7α-hydroxy DHEA and 7α-hydroxy-pregnenolone, with resultant reduction in protection against deleterious effects of cortisol (see US Serial No 09/168,218 from WO97/37664). It is taught that 7α-hydroxy estradiol and precursors thereof, whose synthesis would also be impaired by such enzyme loss, should also be used in treating Alzheimer's disease, although no particular mechanism is implicated for its actions.

It is currently postulated that hormone replacement therapy using estrogens provides for a reduction in the incidence of dementia in women, such as the dementia seen in Alzheimer's disease. Dubal et al. (1999) J. Neurosci 19 (15), p6385-93 report that estradiol mediates neuroprotection in cerebral ischemia in female rats while Zaulyanov et al (1999) 19(6): p705-18 report 133 nM estradiol to protect rat primary cortical neurones from anoxia-reoxygenation induced toxicity. It has also been reported that many estrogens have an antioxidant effect at cellular level, for example in lipid peroxidation assays. However, 7α-hydroxy-estradiol has been reported to be relatively ineffective as an antioxidant in such assays (see Takanashi et al (1995) Biol. Pharm. Bull 18(8) p1120-1125).

7-keto-pregnenolone sulphate has been reported as being a weak inhibitor of NMDA mediated [Ca²⁻], response, decreasing the NMDA response by 12% as compared to 76% by 3α-hydruxy-5β-pregnan-20-one sulphate, Park-Chung *et al*., 1997; Mol Pharm, 52: 1113-1123 and Irwin *at al.,* 1994; J. of Pharm & Ex. Ther. 271: 677-682). WO/97/03677 claims, use of pregnenolone sulphates for inhibiting NMDA receptors for treating inter alia stroke, but does not suggest use of 7-substituted compounds. as compounds selected all exhibit 30% or more decrease in NMDA response.

WO 99/52532 claims use of DHEA and its congeners in general in treating stroke and CNS trauma, but of 54 congeners described as being useful, none is a 7α-hydroxy or 7-keto steroid. WO 98/22113 claims use of estrogens in treatment of ischemia, whether CNS or peripheral, but only exemplifies use of 17α- and 17β-estrogens that are unsubstituted at the 7- position.

The present inventors have now surprisingly determined that 7α-hydroxy DHEAs, 7α-hydroxypregnenolones, and 7α-hydroxyestradiols may be used to treat acute neurodegenerative disease states, such as stroke and head trauma, as well as in the known chronic and anti-glucacorticoid applications already described. Furthermore, far from being a relatively unimportant steroid metabolite physiologically, not only does 7α-hydroxy estradiol have a significant therapeutic role in chronic diseases such as Alzheimer's, it also has a surprising neuroprotective efficacy applicable to treating these acute disorders and disease states. This is particularly surprising, as the inventors do not find this efficacy to be shared by the 7-unsubstituted forms DHEA, pregnenolone or estradiol themselves, which are otherwise thought to be protective in chronic disease.

Thus a first aspect of the present invention provides a medicament for treating a patient in need of therapy for acute cellular degeneration due to metabolic compromise of cells comprising a therapeutically effective amount of a 7α-hydroxy substituted steroid selected from 7α-hydroxyestradiols, 7α-hydroxy-dehydroepiandrosterones, and 7α-hydroxy-pregnenolones.

A second aspect of the invention comprises the use of a 7α-hydroxy substituted steroid selected from 7α-hydroxy-estradiols, 7α-hydroxy-dehydroepiandrosterone and 7α-hydroxy-pregnenolones for the manufacture of a medicament for the treatment of acute cellular degeneration due to metabolic compromise of cells.

A third aspect of the present invention provides a cell function protectant composition characterised in that it comprises a 7α-hydroxy substituted steroid selected from 7α-hydroxy-estradiols, 7α-hydroxy-dehydroepiandrosterones, and 7α-hydroxypregnenolones together with a pharmaceutically acceptable carrier in a form suitable for parenteral administration, particularly in an injectable form, eg suitable for intravenous infusion. Such composition may for example be administered as an aqueous solution through a catheter, with possibility of providing it with other fluids to a patient suffering from stroke, head or spinal trauma, ischaemia or coma.

The compositions of the invention are directed at treating acute neuronal degeneration, such as occurs after head trauma or an ischemic focus within the CNS.

Preferably the steroid in each case is 7α-hydroxy-estradiol (ie. 3β-, 7α-, 17β-estriol, also estra-1,3,5(10)-triene-3β, 7α, 17β-triol) or is 7α-hydroxy-DHEA or carboxylic acid esters of either, eg. 3-acetyloxy. 7α-hydroxy-pregnenolone is found to be less active than these in the present use.

Preferred 7α-hydroxy-estradiol type compounds for the use and composition of the invention are of general formula Ia set out below 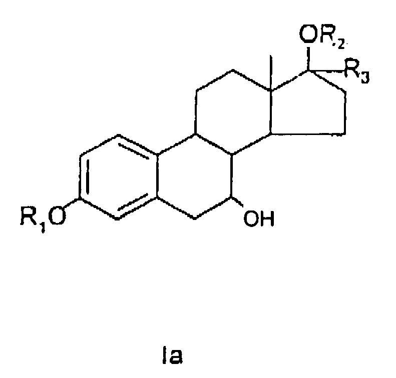 wherein OR₁ and OR₂ each independently represents a free hydroxy group, an ether group or an esterified hydroxy group and R³ is hydrogen, substituted or unsubstituted C₁₋₆ alkyl, C₂₋₆ alkenyl or C₂₋₆ alkynyl, more preferably hydrogen or ethynyl.

Preferably R₁ and R₂ are independently selected from substituted or unsubstituted C₁₋₆ alkyl groups, any such substituents being selected from OH, halogen (F, Cl, Br, I), amino, C₁₋₆ alkylamino, C₁₋₆ dialkylamino, -COOH or -COOR₅ wherein R₅ represents a C₁₋₆ alkyl group which may be unsubstituted or substituted by one of the substituents referred to above; or -OR₁ and -OR² each independently represents an esterified hydroxy group, of the formula R₆COO-, wherein R₆ may be selected from substituted or unsubstituted C₁₋₆ alkyl groups, any such substituents being selected from -OH, halogen (F, Cl, Br, I), amino, C₁₋₆ alkylamino, C₁₋₆ dialkylamino, -COOH or -COOR₅ wherein R₅ represents a C₁₋₆ alkyl group; or
-OR₁ and -OR₂ each independently represents an esterified hydroxy group of formula -OP(OH)₃, or a sulphate group.
or a pharmacologically acceptable salt of such a compound.

Methods of synthesizing derivatives of 7α-hydroxy-estradiols for use in the invention will occur to those skilled in the art on reference to the very considerable body of literature related to synthesis of estrogens GB 1528796 for example details preparation of ethynyl compounds, while etherifications and esterifications all fall within the scope of the skilled person's general knowledge. It will be realised that many of the structures may be reached by subjecting the corresponding 7H compound to the actions of CYP7B or equivalent steroid 7α-hydroxylases (US 5,976,850).

Methods of synthesizing 7α-hydroxy-DHEA, 7-keto-DHEA, 7α-hydroxy-pregnenolone and 7-oxo-pregnenolone and 3-acyl esters (eg 3-acetyloxy) thereof are well documented (see for example US 5,707,983 and US 5,763,433).

By acute cellular degeneration is meant a condition in which cells are losing functionality, temporarily or irreversibly and/or in a process of dying whether through apoptosis or other mechanism.

By metabolic compromise is meant an acute state where a cell is either cut off from sufficient oxygen and/or energy source, eg. a metabolisable molecule that may be used by the cell to produce energy in the form of ATP, NADH and NADPH, most particularly being glucose, or is rendered incapable of using such oxygen or molecule by mechanical injury, eg. resulting in inflammation, increase of intracranial pressure and neuron degeneration eg. apoptosis. In contrast to chronic conditions such as Alzheimer's and Parkinsonism, this compromise is that which is acutely life threatening, with treatment required within hours, or at most a few days, to prevent nerve cell death, whether by apoptosis or other mechanism. It is commonly, but not restricted to, that seen in stroke, eg. ischemia, or head and spinal trauma due to mechanical injury, eg. road accident associated head injury, and coma. Coma may for example be that brought on by an anoxic episode, such as due to drowning, asphyxiation or cessation of breathing or other condition resulting in failure of the cerebral circulation. These are all acute states that result in demonstrable loss of cerebral or peripheral function usually within under 24 hours hours of onset and certainly within 7 days to 3 weeks.

The treatment may be provided prophylactically, such as where a patient is at risk of having an ischemic event, eg. stroke, or may be provided immediately or as soon after the onset of the condition as possible. Other examples of risk states include diagnosis of diseased blood vessels, eg. atherosclerosis or where it is necessary to perform coronary surgery, both benefiting_from treatment for sequelae of ischemic nature as opposed to just anticoagulant therapy.

Compositions may be administered by any conventional method including enteral (for example oral and rectal) or parenteral (for example delivery into the nose or lung or injection into the veins, arteries, brain, spine, peritoneum, muscles or sub-cutaneous region. Most preferably the compounds are administered by enteral (ie. oral), intravenous or transdermal administration

The treatment may consist of a single dose or a plurality of doses over a period of time. The dosage will be determined by the physician but may be between 0.01 mg and 1.0 g/kg/day, for example between 0.1 and 500 mg/kg/day. In terms of dose per square meter of body surface, the compound can be administered at 1.0 mg to 1.5 g per m² per day, for example 3.0-200.0 mg/m²/day.

Whilst it is possible for a compound of the invention to be administered alone, it is preferable to present it as a pharmaceutical formulation, together with one or more acceptable carriers. The formulations may conveniently be presented in unit dosage form. A unit dosage form may comprise for example 2.0 mg to 2.0 g, for example 5.0 mg to 300.0 mg of active ingredient. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or muiti-dose containers, for example scaled ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

### FIGURES

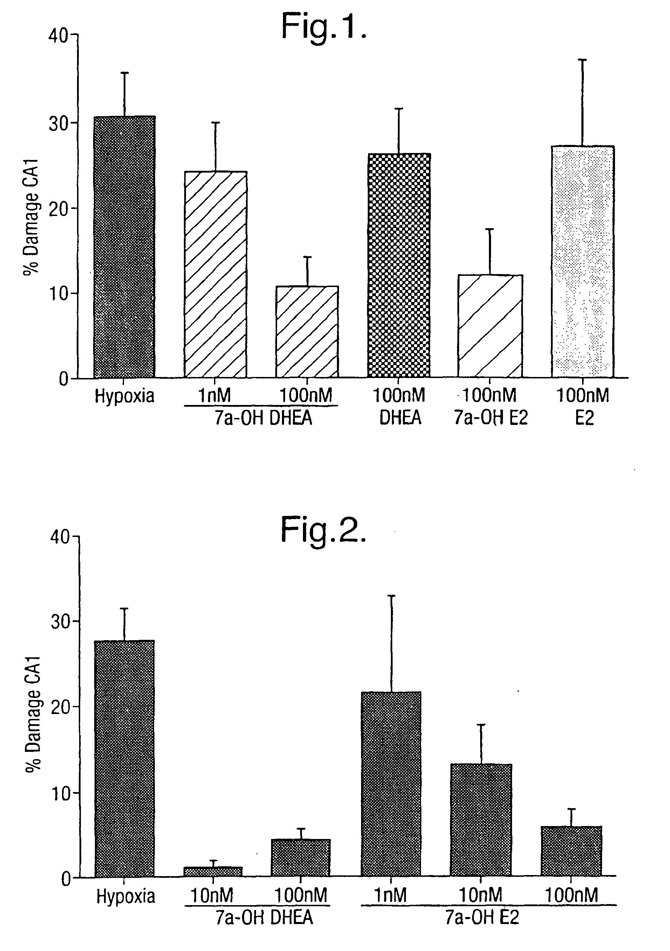

Figure 1: Shows a bar chart indicating the % cells damaged in CA1 after 180minutes oxygen Withdrawal followed by 24 hours of reperfusion, ie. hypoxic challenge, in presence of stated doses of 7α-OHDHEA and 7α-OHE2.
Figure 2: Shows a bar chart illustrating the dose dependent effect of 7α-OHDHEA and 7α-OHE2.
Figure 3: Shows a bar chart illustrating the effect of 7α-OHE2 on cell death in glutamate toxicity assay of primary cortical neurones. Estradiol was found to have no effect.

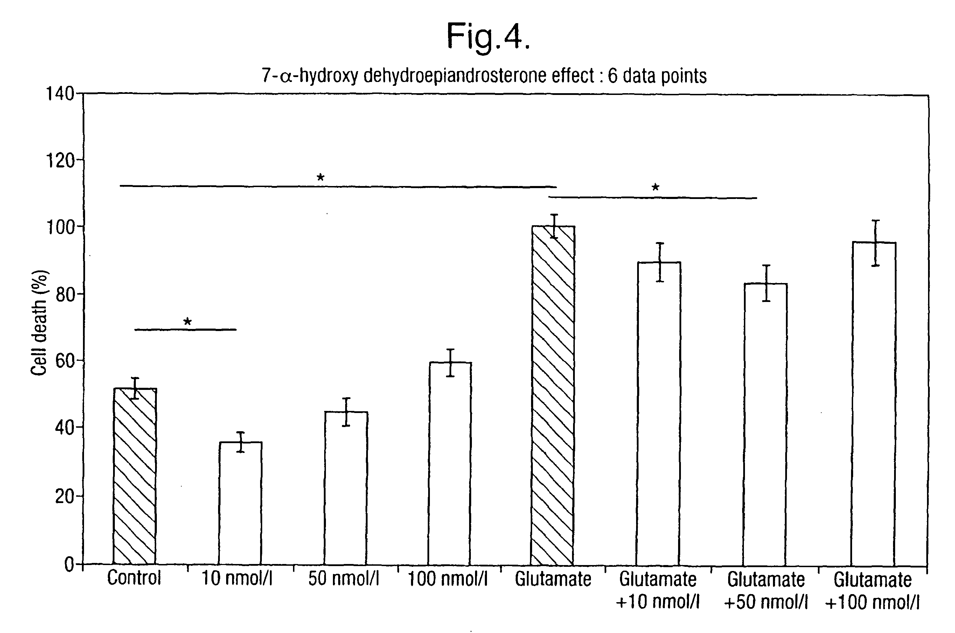

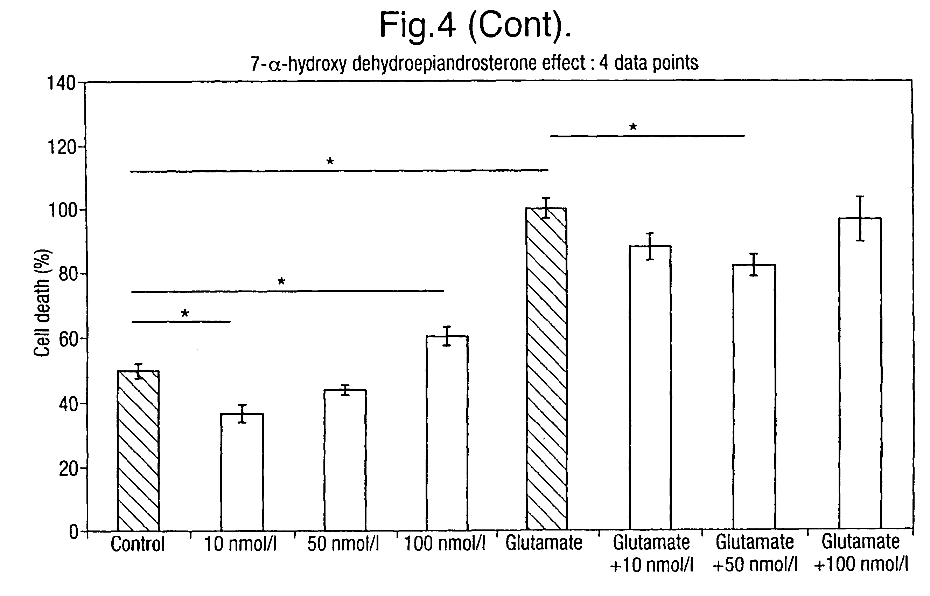

Figure 4: Shows a bar chart illustrating the effect of 7α-OHDHEA 7α-OHE2 on cell death in glutamate toxicity assay of primary cortical neurones.

### EXAMPLE 1 (enabling example)

### The Synthesis of 7α-Hydroxyestradiol (Estra-1,3,5(10)-triene 3,7α,17β-triol)

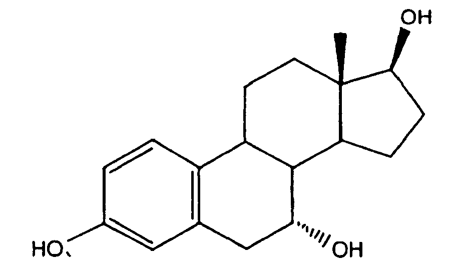

The title compound can be prepared by a two step process involving epoxidation of estra-1,3,5(10),6-tetraene-3,17β-diol diacetate (7), followed by opening of the epoxide ring, with removal of the acetate groups, by reaction with lithium aluminium hydride. The following protocol was used due to temporary problems in sourcing estratetraene (7), making it necessary to undertake 6 extra steps to prepare (7) from β-estradiol (1).

β-Estradiol (1) was converted to the diacetate (2) in 94% yield by reaction with acetic anhydride heated under reflux for 1 hour in pyridine. The diacetate (2) was oxidised with chromium (VI) oxide in acetic acid-water, followed by purification by chromatography and recrystallisation from ethanol-water to afford 6-keto-estradiol diacetate (3) in 18% yield (23% yield for small scale reaction)².

The ketone (3) was reacted with tosylhydrazide heated under reflux for 6h in ethanol to produce the tosylhydrazone (4) in 83% yield. The acetate groups were removed in quantitative yield by reaction at room temperature with methanolic potassium hydroxide. The tosylhydrazone diol (5) was then converted into the estratetraene (6) in 36% yield after purification by flash chromatography (54% yield for small scale reaction), by a Shapiro reaction in which a solution of (5) in dry tetrahydrofuran was treated with methyllithium at 0°C and then allowed to warm to room temperature. Reaction of the diol (6) with acetic anhydride heated under reflux for 1h in pyridine gave the diacetate (7) in 91 % yield³.

The estratetraene (7) was epoxidised by treatment in ether-tetrahydrofuran with an excess of monoperphthalic acid⁴ at 5°C for 1 day. An 85% yield of the 6α,7α-epoxide (8) was obtained following purification by flash chromatography. The synthesis was completed by reaction of the epoxide diacetate (8) with lithium aluminium hydride heated under reflux in tetrahydrofuran. After purification by flash chromatography, an 85% yield of 7α-hydroxyestradiol (9) was obtained⁵. The ¹H NMR, IR and mass spectra of (9) were consistent with its structure

The Scheme that follows illustrates the synthetic pathway to the title compound.

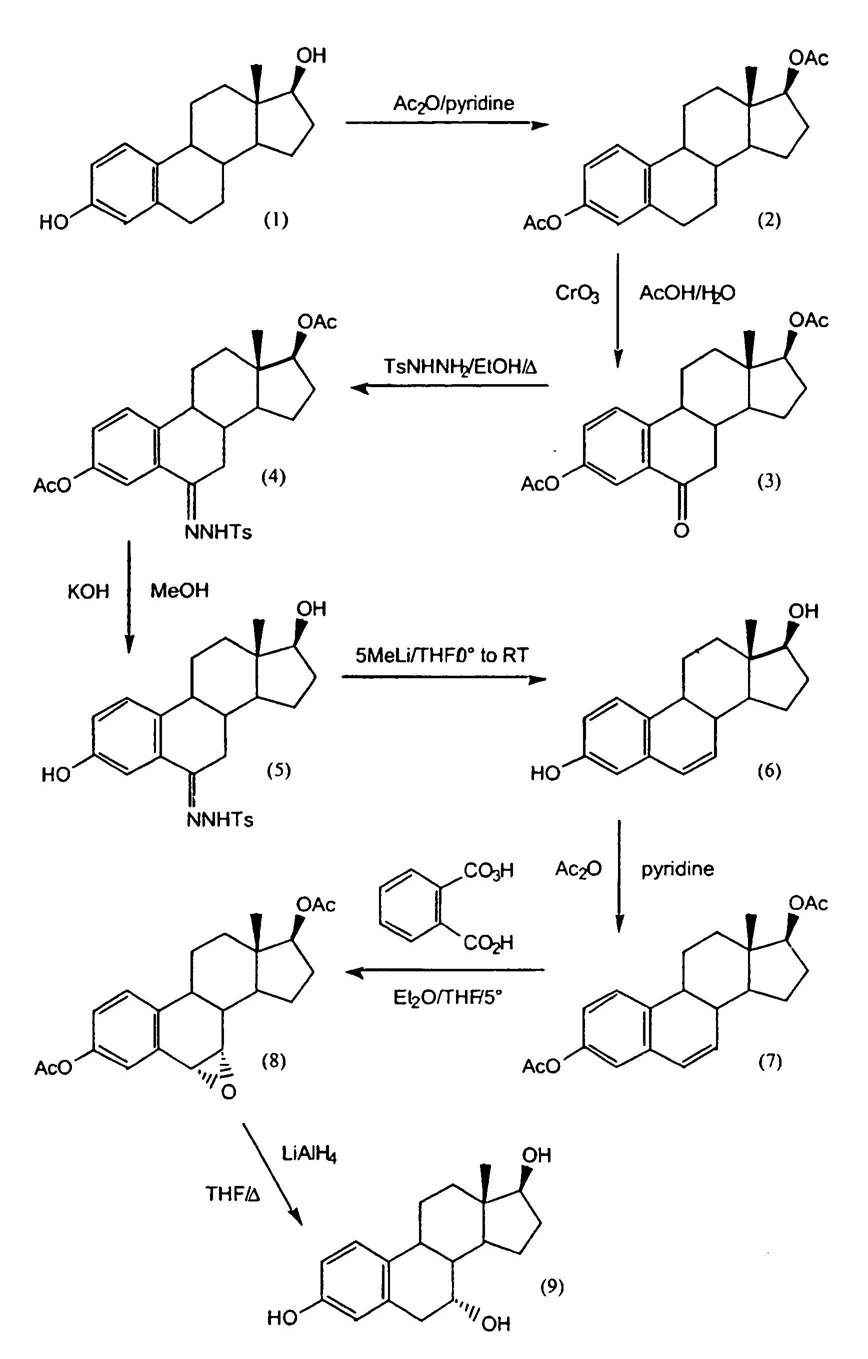

### 1) General.

For thin layer chromatography (TLC) aluminium foil plates pre-coated with silica gel were employed. The solvent systems used for elution of the plates were all mixtures of ethyl acetate and petroleum spirit (EtOAc content indicated), except for the final step where 8% methanol in dichloromethane was used. The results of elution of the TLC plates were visualised under UV light and with phosphomolybdic acid stain: step 1, 33% EtOAc: (1) R_{f} 0.3. (2) R_{f} 0.9; step 2. 20% EtOAc: (2) Rr 0.8, (3) R_{f} 0.6; step 3, 20% EtOAc: (3) Rr 0.6, (4) R_{f} 0.4; step 4, 33% EtOAc: (4) R_{f} 0.6, (5) R_{f} 0.4; step 5, 40% EtOAc: (5) R_{f} 0.5, (6) R_{f} 0.2; step 6, 40% EtOAc: (6) R_{f} 0.2, (7) R_{f} 0.6; step 7, 25% EtOAc: (7) R_{f}0.5, (8) R_{f}0.3; step 8, 8% McOH: (8) R_{f}0.85, (9) R_{f}0.15

### 2) Experimental procedures⁶

Caution : Some steroids are carcinogens and/or teratogens. Appropriate precautions should be taken to avoid exposure to these compounds as well as the toxico/corrosive reagents which are used.

### Preparation of Estra-1,3,5(10)-triene-3,17β-diol Diacetate (2)

Acetic anhydride (70 ml) was added to a stirred solution of β-estradiol (20g, 73.4 mmol) in dry pyridine (280 ml) and the mixture was heated under reflux for 1h. After standing overnight, the mixture was poured into ice-water (1000 ml) with stirring. The solid was collected by filtration and recrystallised from ethanol-water. The product was obtained as off-white lustrous platelets and dried *in vacuo* (*ca.* 60°C) for 2 hours. First crop 23.6g (90%), m.p. 128-129°C (lit² 126-128°C); second crop 1.1 g (4%), m.p. 126-128°C. Both crops homogeneous and identical by TLC. Total yield 24.7 g (94%).

### Preparation of 3,17β-Diacetoxyestra-1,3,5(10)-trien-6-one (3)

A solution of chromium (V1) oxide (16.9 g, 169 mmol) in acetic acid (102 ml) and water (14 ml) was added to a stirred solution of estra-1,3,5(10)-triene-3,17β-diol diacetate (2) (20.0g, 56.1 mmol) in acetic acid (70 ml). The mixture was stirred at room temperature for 24 hours, poured into water (1000 ml) and extracted with ether (2x 750 ml, 2x 400 ml). The ether solution was washed with sat. sodium hydrogen carbonate (200 ml portions; caution: vigorous CO₂ evolution) until the aqueous phase turned pink. Washing was then done with a 3:1 mixture of 1 M sodium carbonate and sat. sodium hydrogen carbonate (300 ml) and with water (2x 200 ml), and the solution was dried (MgSO₄). The solvent was evaporated to afford the crude product (16g) as a yellow oil which was chromatographed on silica (300g) eluting with a solvent polarity gradient (0% to 1% ethyl acetate in dichloromethane). Product fractions were combined and evaporated, and the residue was recrystallised from ethanol with a small amount of water added. The product was obtained as an off-white solid and dried *in vacuo* (*ca.* 60°C) for 2h. Yield 2.95g (14%), m.p. 171-175°C (lit² 173-175°C). Evaporation of the mother liquor afforded a second batch of product, containing minor impurities, as a yellow gum (0.77g, 4%). Total yield 3.72g (18%).

### Preparation of 3,17β-Diacetoxyestra-1,3,5(10)-trien-6-one Tosylhydrazone (4)

A stirred solution of 3,17β-diacetoxyestra-1,3,5(10)-trien-6-one (3) (3.68g, 9.93 mmol) and toluene-4-sulphonylhydrazide (3.68g, 19.8 mmol) in ethanol (230 ml) was heated under reflux for 6 hours. After standing overnight, the yellow solid was collected by filtration and dried *in vacuo* (*ca.* 60°C) for 2h. Yield 3.96g (74%). Evaporation of the mother liquor and chromatographic clean-up eluting with ethyl acetate - iso-hexane (gradient of 1:3 to 1:2) afforded more of the product (0.46g, 8%). Total yield 4.42g (83%).

### Preparation of 3,17(β-Dihydroxyestra-1,3,5(10)-trien-6-one Tosylhydrazone (5)

To a stirred suspension of 3,17β-diacetoxyestra-1,3,5(10)-trien-6-one tosylhydrazone (4) (3.94g, 7.31 mmol) in methanol (330 ml) was added methanolic 1M potassium hydroxide (23 ml). The mixture was stirred at room temperature over a weekend. The solvent was evaporated and the residue was partitioned between ethyl acetate (450 ml) and half-saturated brine (200 ml) acidified with 2 M hydrochloric acid (12 ml). The organic layer was separated, washed with half-saturated brine (150 ml) and dried (MgSO₄). The solvent was evaporated and the residue azeotropically dried with benzene, followed by removal of residual solvent *in vacuo* (*ca.* 60°C) to afford the product as a yellow solid (3.35g, 100%).

### Preparation of Estra-1,3,5(10),6-tetraene-3,17β-diol (6)

1.4 M Methyl-lithium ethereal solution (26.5 ml, 37.1 mmol) was added, dropwise via a syringe, to a stirred solution of 3,17β-dihydroxyestra-1,3,5(10)-trien-6-one tosylhydrazone (5) (3.25g, 7.1 mmol) in anhydrous tetrahydrofuran (550 ml) at 0° under argon. The mixture was allowed to warm gradually to room temperature and further portions of 1.4 M methyllithium solution (6.0 ml, 8.4 mmol) were added after 4 and 18 hours. The mixture was then poured into ice (*ca.* 400 ml), acidified by the addition of 2 M hydrochloric acid (25 ml) and extracted with ethyl acetate (2x 450 ml). The ethyl acetate solution was washed with sat. sodium hydrogen carbonate solution (3x 250 ml) and water (2x 150 ml), and dried (MgSO₄). The solution was concentrated and silica (6g) was added, before evaporation to dryness. The residue was placed on the top of a flash chromatography column (85g silica) which was eluted with *iso*-hexane and then ethyl acetate - iso-hexane (gradient of 1:3 to 1:2). Product fractions were combined and evaporated, and residual solvent was removed *in vacuo (ca.* 60°C) for 1h to afford the product as an off-white solid (0.70g, 36%).

### Preparation of Estra-1,3,5(10),6-tetraene-3,17β-diol Diacetate (7)

Acetic anhydride (2.8 ml) was added to a stirred solution of estra-1,3,5(10),6-tetraene-3,17β-diol (6) (0.80g, 2.96 mmol) in dry pyridine (12 ml) and the mixture was heated under reflux for 1 hour. When cool, the mixture was poured into ice-water (40 ml) with stirring. The solid was collected by filtration, washed with cold water and dried *in vacuo* (*ca.* 60°C) for 1 hour to afford the product as a beige solid (0.95, 91%).

### Preparation of Monoperphthalic Acid

A suspension of phthalic anhydride (10.0g, 67.5 mmol) and sodium perborate (14.9g, 96.8 mmol) in water (45 ml) was stirred at 0°C for 2 hours. The mixture was filtered, the filtrate was acidified with ice-cold 30% sulphuric acid and extracted with ether (3x 25 ml). Emulsion formation was a problem, so some brine was added. The combined ethereal solution was washed with brine and dried (MgSO₄). The ethereal solution of monoperphthalic acid was filtered and stored at 5°C. It should be *ca.* 0.7M monoperphthalic acid.

### Preparation of 6α,7α-Oxido-estra-1,3,5(10)-triene-3,17β-diol Diacetate (8)

Ethereal monoperphthalic acid solution (14 ml) was added to a stirred solution of estra-1,3,5(10),6-tetraene-3,17β-diol diacetate (7) (0.84g, 2.37 mmol) in 1:1 ether-tetrahydrofuran (24 ml) at 0°C. The solution was transferred to a refrigerator and left to stand at 5°C for 1 day. Ether (50 ml) was added, the solution was washed with sat. sodium hydrogen carbonate solution (20 ml, 2x 10 ml), dried (MgSO₄) and evaporated. The crude product was purified by chromatography on silica (70g) eluting with ethyl acetate - iso-hexane (1:4.5). Product fractions were combined and evaporated, and residual solvent was removed *in vacuo* to afford the product as a white solid (0.75g, 85%).

### Preparation of Estra-1,3,5(10)-triene-3,7α,17β-triol (9)

A solution of 6α,7α-oxido-estra-1,3,5(10)-triene-3,17β-diol diacetate (8) (0.80g, 2.16 mmol) in anhydrous tetrahydrofuran (45 ml) was added dropwise to a stirred suspension of lithium aluminium hydride (1.84g, 48.5 mmol) in anhydrous tetrahydrofuran (185 ml) and the mixture was then heated under reflux under argon for 18 hours. Excess lithium aluminium hydride was destroyed by the cautious addition of water at 0°C. 2M Hydrochloric acid (100 ml) and water (50 ml) were added to dissolve the solids giving a greenish opaque lower (aqueous) phase. The mixture was extracted with ethyl acetate (2x 250 ml, 150 ml) and the ethyl acetate solution was dried (MgSO₄) and evaporated to afford a solid residue which was dissolved in methanol. Silica (3g) was added, the mixture was evaporated to dryness and the residue placed on top of a flash chromatography column which was eluted with methanol-dichloromethane (gradient of 6% to 15% MeOH). Product fractions were combined and evaporated, and the residue was dissolved in ethyl acetate with a small amount of added methanol and then filtered. The solvent was evaporated, and residual solvent was removed *in vacuo* (*ca.* 110°C) for 2 hours, to afford the product as an off-white solid (0.53g, 85%), m.p. 254-257°C (lit⁵ 259-260°C).

### References

1. Steraloids Inc., Wilton, New Hampshire, USA. estra-1,3,5(10),6-tetraene-3,17β-diol diacetate (cat. No. E475).
2. P.D.G. Dean, D. Exley and M.W. Johnson, ***Steroids,*** 1971, **18**, 593.
3. J.C. Gill, B.A. Marples and J.R. Traynor, ***Synth. Commun.,*** 1989, **19**, 155.
4. L.F. Fieser and M. Fieser, "Reagents for Organic Synthesis", Vol. 1, 819.
5. J. Iriarte, H.J. Ringold and C. Djerassi, ***J. Am. Chem.** Soc.,* 1958, **80**, 6105.
7α-hydroxy-DHEA, 7α-hydroxy-pregnenolone, 7-oxo-DHEA and 7-oxo-pregnenolone and 3-acetyl derivatives thereof are readily synthesizable using the method of Lardy et al (See US 5,707,983) and analogous methods.

### EXAMPLE 2

### Effect of 7α-hydroxy steroids on hypoxic challenge of hippocampal slices.

Organotypic hippocampal slice cultures were prepared using the basic method of Pringle *et al* (1997) modified as follows:

Wistar rat pups (8-11 days old) were decapitated and the hippocampus rapidly dissected into ice-cold Goy's balanced salt solution supplemented with 4.5 mg/ml glucose. Slices were separated and plated into Millicell CM culture inserts (4 per well) and maintained at 37°C/5%CO₂ for 14 days. Maintenance medium consisted of 25% heat-inactivated horse serum, 25% Hank's balanced salt solution (HBSS) and 50% minimum essential medium with added Earle's salts (MEM) supplemented with 1 mM glutamine and 4.5 mg/ml glucose.

Experimental hypoxia was performed as described previously (Pringle *et al.,* (1996) Stroke 27, p2124-2130, (1997) Brain Research 755-46). Briefly, cultures were transferred to serum free medium (SFM - 75% MEM, 25% HBSS supplemented with 1 mM glutamine and 4.5 mg/ml glucose) containing 5 µg/ml of the fluorescent exclusion dye propidium iodide (PI). Cultures were allowed to equilibrate in SFM for 60 minutes prior to imaging. PI fluorescence was detected using a standard Leica inverted microscope fitted with a rhodamine filter set (Leica N2). Any cultures in which PI fluorescence was detected at this stage were excluded from further study. Hypoxia was induced by transferring cultures to SFM (+PI) which had been saturated with 95%N₂/5%CO₂. Culture plates (without lids) were then sealed into an airtight chamber in which the atmosphere was saturated with 95%N₂/5%CO₂ by continuously blowing through gas at 1000 ml/min for ten minutes before being sealed and placed in the incubator for 170 mins (total time of hypoxia was therefore 180 mins). At the end of the hypoxia period cultures were returned to normoxic SFM containing PI and placed back in the incubator for 24 hours.

Neuronal damage was assessed as described previously (Pringle *et al*., 1996, 1997) using NIH Image 1.60 running on an Apple IIsi computer. Images were captured using a COHU monochrome camera and saved onto optical disk for offline analysis using NIH image. Light transmission images were captured prior to the addition of drugs, and PI fluorescence images recorded at the end of the 24 hour post-hypoxia recovery period. The arcas of the CA1, CA3 and dentate gyrus (DG) cell layers were determined from the transmission image. The area of PI fluorescence in each of the cell layers was measured using the density slice function within NIH Image, and neuronal damage expressed as the percentage of the regional area in which PI fluorescence was detected above background.

Steroid compounds were prepared by making an initial 1 mg/ml solution in ethanol and further diluting down in SFM. Compounds were included in the cultures for 45 minutes prior to hypoxia, during the hypoxic episode and during the post-hypoxic recovery period. Control experiments consisted of cultures treated with vehicle alone.

### Results:

24 hours after 180 minutes of hypoxia PI fluoresence was detectable in 27.6±3.7% of the CA1 pyramidal cell layer (n=38). 7α-hydroxy-estradiol (7OHE2) was found to be highly neuroprotective in a concentration dependent manner. In cultures treated with 100nM 7OHE2 damage was observed in 5.9±2.1% of CA1 (n=22, p<0.001 vs hypoxia). At 10 nM however, the degree of neuroprotection was significantly reduced with damage detected in 13.0±4.7% of CA1 (n=8, p=NS vs hypoxia) and at 1nM the damage was indistinguishable from hypoxia controls (21.5±11.3%, n=7, p=NS vs hypoxia).

7α-hydroxy-DHEA reduced neuronal death in CA1 to 4.3±1.3% (n=23, p<0.001 vs hypoxia). No loss of neuroprotective efficiency was observed when the concentration was reduced to 10 nM (CA1 damage 1.1±0.9%, n-=2, p<0.001 vs hypoxia).

7α-hydroxy-pregnenolone was neuroprotective at 100 nM with PI fluorescence occurring in 11.6±4.0% of CA1 (n=16, p<0.005 vs hypoxia). This is significantly less potent than for the other two test compounds.

### EXAMPLE 3

### Effect of 7α-hydroxy steroids on glutamate induced neuronal cell death of primary cortical cultures.

L-glutamate is the major excitatory amino acid in the brain of vertebrates and mediates physiological responses such as synaptic plasticity and long term potentiation. However, overstimulation of glutamate receptors, particularly of the NMDA subtype, is believed to initiate cellular processes leading to neurodegenerative conditions. *In vitro* a brief exposure to glutamate causes neuronal death mainly by hyperstimulating NMDA receptors present on most neurones causing calcium ion influx.

### Materials and Methods:

Rat cortical neurons were cultured by modification of the methods of Dichter (1973) Brain Res 149, p279-293 and Durkin et al (1996) J. Neurochem 66, p951-962. A female Wistar rat (Janvier, Le Genest-St-Isle, France: ref RJ WAF151) of 17 days gestation was killed by cervical disclocation, the foetuses removed on embryonoic day 17 and brains removed and placed in ice-cold calcium/magnesium free Hanks balanced salt solution (HBSS: Gibco Life Technologies, Cergy-Pontoise, France: ref 14170-088 batch 302790). Each cortex was dissected and meninges were carefully removed.

The cortical neurons were dissociated by trypsinization for 5 minutes at 37°C (trypsin: 0.5mg/ml: Difco, LPCR, Strasbourg: ref 0152-14 batch 128856JC) and the reaction stopped by addition of HBSS containing calcium and magnesium (HBSS+ Gibco rcf 24020-091; batch 3010598) with 10% of fetal bovine serum FBS; Gibco; ref 1027-098; batch 40Q1681K and 1 mg/ml DNase 1 (Boehringer Mannheim, Meylan, France; ref 1284938; batch 14880300).

The suspension was triturated with a 10 ml pipette (stripette Costar, Dutscher, Brumath, France) and was added in a centrifuge tube (Tube 50 ml Coming, Dutscher) containing 20 ml HBSS with 3.5% of BSA (Sigma, L'Isle D'Abeau Chesnes, ref. A6003, batch 29H7606) and centrifuged at low speed (150 x g) for 10 min (apparatus Sigma 2-15, Bioblock, Illkirch, France).

The cells were resuspended in Dulbecco's Modified Eagle Medium (DMEM; Gibco; ref. 41965-039; batch 3032129), containing 20% FBS. Viable cells were quantified and plated at 35,000/well in 96 well plates (Nunclon, Gibco) previously coated with poly-L-lysine (0.01 mg/ml, France; ref. P5899; batch 28H8504). Cells were allowed to adhere 2 h and maintained in a humidified incubator at 37°C in 5% CO₂-95% air atmosphere.

Culture medium was then changed to a medium consisting of DMEM, 10% heat-inactivated FBS, 10% heat-inactived horse serum (Gibco, ref. 16505-080; batch 3030347) and 1% L-Glutamine (Gibco, ref. 25030-024; batch 3031499).

Since the cultures were mixed populations containing both neurons and glial cells, to minimize glial growth, the cultures were treated for 2 days with 15 µg/ml of 5-fluoro-2'-deoxyuridine (Sigma; ref. F8791; batch 97H4048) and 35 µg/ml of uridine (Sigma, ref. U3003; batch 18H 11965) on day 4 of culture.

Following exposure to mitotic inhibitors, one-half of the medium was removed and replaced with growth medium consisting of 89% DMEM, 10% horse serum and 1% L-glutamine.

On day 10 of culture, the medium was changed to DMEM/HamF12 (Gibco, ref. 21331-020; batch 23243) supplemented with 12.5 mg/ml glutamine (Gibco ref 15750-032. batch 3009462), 20 µg/ml transferrin (Sigma, ref. T1428; batch 97H0257), 5 µg/ml insulin (Sigma, ref. 16634; batch 96H04865), 33 mM glucose (Sigma. ref. G7021; batch 75H07293), 100 µM putrescin (Sigma. ref. P7505; batch 95H0814), 20 nM progesterone (Sigma, ref. P8783; batch 92H01436), 1% glutamine, 7 mM NaHCO₃ (Prolabo, LPCR, ref. 27776365; batch 92329), 30 nM selenite sodium (Sigma; ref. S9133; batch 107H9503) and 1 nM estradiol (Sigma; ref. E2758; batch 65H07865).

Glutamate toxicity experiments were performed after 13 days in culture (Glutamic acid; Sigma, ref. G6904; batch 18H11381).

### Protection

On day 12, twenty-four hours before glutamate exposure, cultures were washed with DMEM and placed in medium DMEM/Ham12 with supplements but without estradiol.

The reference compounds, estradiol (Sigma, ref. E8875; batch 98H40953) and DHEA (Sigma, ref. D4000; batch 97H0301), and test compound were added at concentrations of 2, 10, 50 and 100 nM. Human FGF-basic (Tebu, Le Perray-en-Yvelines, France; ref. 100-18B; batch 107R0806 E118) was included as an additional reference and was tested at 5, 10 and 20 ng/ml. All compounds were dissolved at 10 mM in ethanol 100% (LPCR, ref. 20821467; batch J109), diluted 10,000 fold in DMEM and stocked at -20°C.

The effect of the test molecule on survival of cultured neurones was investigated alone and in intoxication conditions. The effect of the ethanol was also tested at the highest concentration used for dilution of the test molecules (100 nM = 0.001 % ethanol). In the vehicle conditions used, ethanol did not demonstrate an effect on the survival of cultured cortical neurones.

### Glutamate toxicity

On day 13, cultures were exposed to glutamate 0. 50, 100, 150. 200, 250, 300, 400 and 500 µM for 10 min at 37°C in DMEM/hamF12 with supplement. The effects of the 2 reference compounds and the test compound was investigated in the presence of glutamate.

After the incubation period, the cultures were washed with DMEM and placed in DMEM/Ham12 with supplement either in the absence or presence of the compound to evaluate their post intoxication effects.

### Lactate dehydrogenase

Glutamate toxicity was evaluated by measuring lactate dehydrogenase (LDH) activity released in the media 24 hours after glutamate exposure, using the CytoTox 96 non-radioactive kit (Promega, Charbonniéres, France, ref. G1780; batches 109578 and 106244L) and quantitated by measuring wavelength absorbance at 450 nm (Labsystems Multiskan Bichromatic, Cergy Pontoise, France).

### Data presentation

For glutamate intoxication, data are presented as percentage of surviving neurones with the non-treated control expressed as 100%.

The results are presented as histograms of percentage cell death. Each experiment is presented with 2 figures: showing either the means of 6 values or the means of 4 values (highest and lowest data points being excluded).

### Results

Exposure to L-glutamate on day 13 decreased the number of surviving cortical neurones in culture. The neurotoxic action of L-glutamate was concentration-dependent between 0 and 150 µM. with a robust and stable neurotoxic effect (40% of neurones surviving) obtained at doses of 200 µM and greater. 200 µM was selected as the dose of glutamate for subsequent experiments.

The reference compounds, estradiol and DHEA tested at doses of 0, 50 and 100 nM, while failing to protect cortical neurones from glutamate neurotoxicity demonstrated effects in their own right. Both estradiol (10 nM) and DHEA (10 and 50 nM) appeared to protect significantly (p < 0.05) against normal cell death. However, estradiol at 100 nM significantly increased cell death relative to that detected in the control.

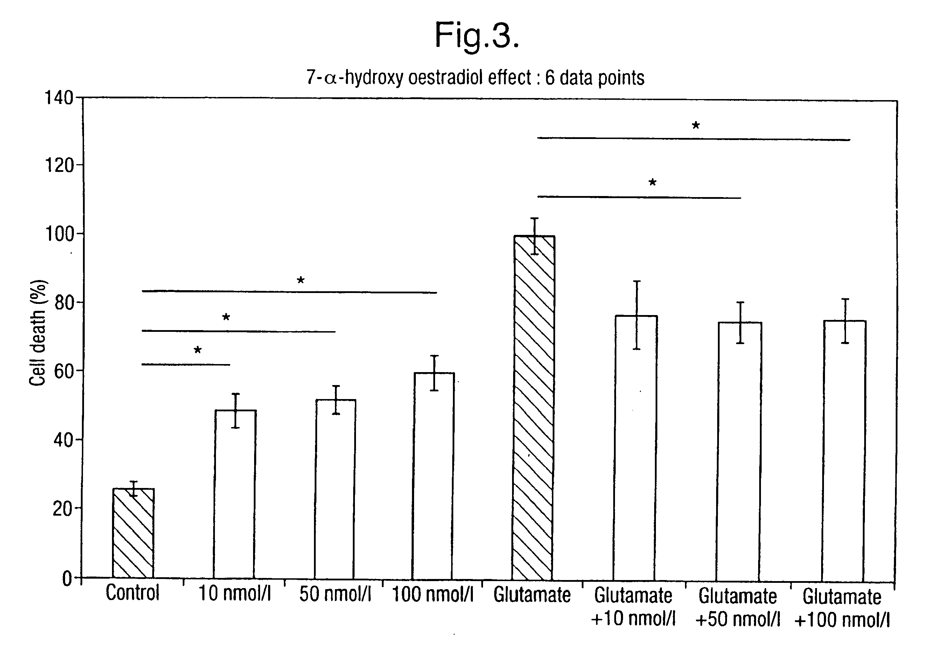

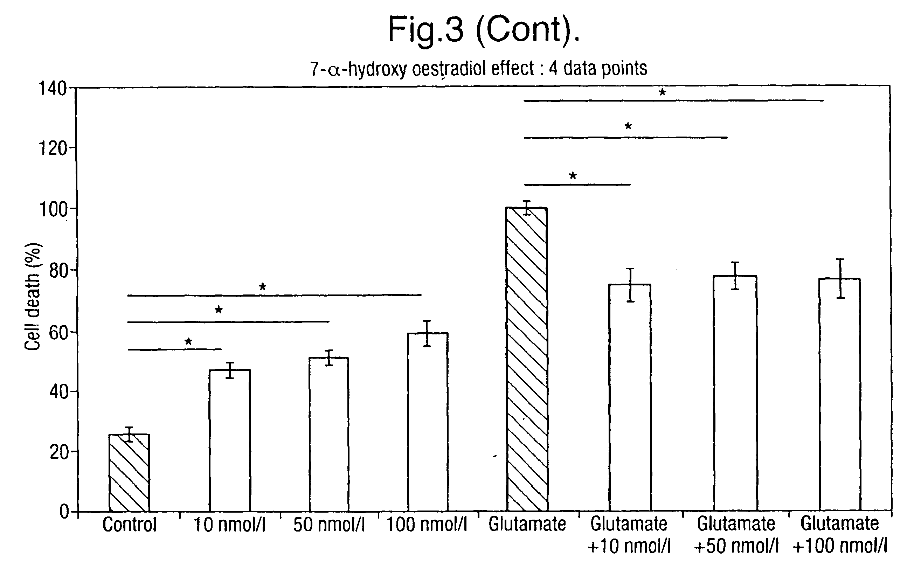

In contrast 7α-hydroxy-estradiol provided up to 20% less cell death at doses of 10 to 100 nM in presence of glutamate See Figure 3. 7α-hydroxy-DHEA at 10 nM significantly decreased normal cell death and provided up to 20% less cell death in the presence of glutamate. 7α-hydroxy-pregnenolone provided up to 10% less cell death at the same dose.

## Claims

1. Use of a 7α-hydroxy-substituted steroid selected from 7α-hydroxy-estradiols, 7α-hydroxy-dehydroepiandrosterones and 7α-hydroxy-pregnenolones for the manufacture of a medicament for the treatment of one or more of stroke, ischaemia, coma and head or spinal trauma.

2. The use of claim 1 **characterised in that** the stroke, ischaemia, coma and head or spinal trauma results in degeneration of cells of the central or peripheral nervous system.

3. The use of claims 1 and 2 **characterised in that** the stroke, ischaemia, coma and head or spinal trauma is an acute state wherein a cell is either cut off from sufficient oxygen and/or molecules which it can use as an energy source or is rendered incapable of using such oxygen or molecule by mechanical injury.

4. The use of claims 1 and 2 **characterised in that** the stroke, ischaemia, coma and head or spinal trauma results in inflammation, increase of intracranial pressure and neuron degeneration or apoptosis resulting in loss of cerebral or peripheral function within 7 days of onset.

5. The use of any preceding claim **characterised in that** the treatment is provided prophylactically.

6. The use of claims 1 to 3 **characterised in that** the 7α-hydroxy-steroid is an estradiol (i.e. an estra-1,3,5(10)-triene-3,7α,17β-triol) of general formula Ia. 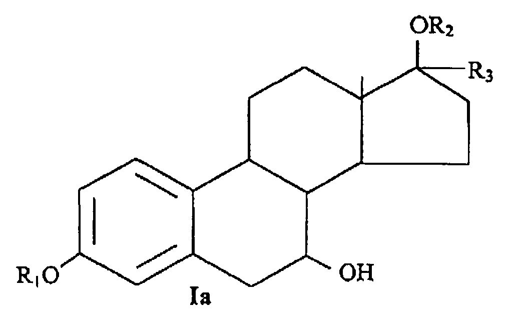 wherein OR₁ and OR₂ each independently represents a free hydroxy group, an ether group or an esterified hydroxy group and R₃ is hydrogen, substituted or unsubstituted C₁₋₆ alkyl, C₂₋₆ alkenyl or C₂₋₆ alkynyl.

7. The use of claim 6 **characterised in that** R₁ and R₂ are independently selected from substituted or unsubstituted C₁₋₆ alkyl groups, any such substituents being selected from OH, halogen (F, Cl, Br, I), amino, C₁₋₆ alkylamino, C₁₋₆ dialkylamino, -COOH or -COOR₅ wherein R₅ represents a C₁₋₆ alkyl group which may be unsubstituted or substituted by one of the substituents referred to above; or -OR₁ and -OR₂ each independently represents an esterified hydroxy group, of the formula R₆COO- wherein R₆ may be selected from substituted or unsubstituted C₁₋₆ alkyl groups, any such substituents being selected from -OH, halogen (F, Cl, Br, I), amino, C₁₋₆ alkylamino, C₁₋₆ dialkylamino, -COOH or -COOR₅ wherein R₅ represents a C₁₋₆ alkyl group; or
-OR₁ and -OR₂ each independently represents an esterified hydroxy group of formula -OP(OH)₃, or a sulphate group,
or a pharmacologically acceptable salt of such a compound.

8. The use of claims 1 to 5 **characterised in that** the steroid is a 7α-hydroxy-DHEA or 3-acyloxy esters thereof.

## Patentansprüche

1. Verwendung eines 7α-hydroxysubstituierten Steroids, ausgewählt aus 7α-Hydroxyestradiolen, 7α-Hydroxydehydroepiandrosteronen und 7α-Hydroxypregnenoloncn, für die Herstellung eines Medikaments für die Behandlung von einem oder mehreren von Schlaganfall, Ischämie, Koma und Schädel- oder Rückenmarkstrauma.

2. Verwendung nach Anspruch 1, **dadurch gekennzeichnet, daß** Schlaganfall, Ischämie, Koma und Schädel- oder Rückenmarkstrauma zu Degeneration von Zellen des zentralen oder peripheren Nervensystems führen.

3. Verwendung nach den Ansprüchen 1 und 2, **dadurch gekennzeichnet, daß** Schlaganfall, Ischämie, Koma und Schädel- oder Rückenmarkstrauma ein akuter Zustand sind, wobei eine Zelle entweder von ausreichendem Sauerstoff und/oder Molekülen, welche sie als Energiequelle verwenden kann, abgeschnitten ist oder durch mechanische Verletzung unfähig gemacht ist, derartigen Sauerstoff oder derartige Moleküle zu verwenden.

4. Verwendung nach den Ansprüchen 1 und 2, **dadurch gekennzeichnet, daß** Schlaganfall, Ischämie, Koma und Schädel- oder Rückenmarkstrauma zu Entzündung, Zunahme des intrakraniellen Drucks und Neuronendegeneration oder Apoptose führen, die zum Verlust von zerebraler oder peripherer Funktion innerhalb von 7 Tagen nach dem Beginn führen.

5. Verwendung nach einem vorhergehenden Anspruch, **dadurch gekennzeichnet, daß** die Behandlung prophylaktisch bereitgestellt wird.

6. Verwendung nach den Ansprüchen 1 bis 3, **dadurch gekennzeichnet, daß** das 7α-Hydroxysteroid ein Estradiol (d.h. ein Estra-1,3,5(10)-trien-3,7α,17β-triol) der allgemeinen Formel Ia 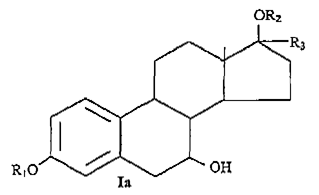 ist, wobei OR₁ und OR₂ jeweils unabhängig eine freie Hydroxygruppe, eine Ethergruppe oder eine veresterte Hydroxygruppe darstellen und R₃ Wasserstoff, substituiertes oder unsubstituiertes C₁₋₆-Alkyl, C₂₋₆-Alkenyl oder C₂₋₆-Alkinyl ist.

7. Verwendung nach Anspruch 6, **dadurch gekennzeichnet, daß** R₁ und R₂ unabhängig aus substituierten oder unsubstituierten C₁₋₆-Alkylgruppen ausgewählt sind, wobei alle derartigen Substituenten aus OH, Halogen (F, Cl, Br, I), Amino, C₁₋₆-Alkylamino, C₁₋₆-Dialkylamino, -COOH oder -COOR₅ ausgewählt sind, wobei R₅ eine C₁₋₆-Alkylgruppe darstellt, welche unsubstituiert oder mit einem von den vorstehend in Bezug genommenen Substituenten substituiert sein kann; oder -OR₁ und -OR₂ jeweils unabhängig eine veresterte Hydroxygruppe mit der Formel R₆COO- darstellen, wobei R₆ aus substituierten oder unsubstituierten C₁₋₆-Alkylgruppen ausgewählt sein kann, wobei alle derartigen Substituenten aus OH, Halogen (F, Cl, Br, I), Amino, C₁₋₆-Alkylamino, C₁₋₆-Dialkylamino, -COOH oder -COOR₅ ausgewählt sind, wobei R₅ eine C₁₋₆-Alkylgruppe darstellt; oder -OR₁ und -OR₂ jeweils unabhängig eine veresterte Hydroxygruppe der Formel -OP(OH)₃ oder eine Sulfatgruppe darstellen,
oder eines pharmakologisch verträglichen Salzes einer derartigen Verbindung.

8. Verwendung nach den Ansprüchen 1 bis 5, **dadurch gekennzeichnet, daß** das Steroid ein 7α-Hydroxy-DHEA oder 3-Acyloxyester davon sind.

## Revendications

1. Utilisation d'un 7α-hydroxy-stéroïde substitué choisi dans le groupe constitué des 7α-hydroxy-estradiols, 7α-hydroxy-deshydroepiandrosterones et 7α-hydroxy-hydroprégnénolones pour la fabrication d'un médicament destiné au traitement d'un ou plus des accidents cérébro-vasculaire, ischémie, coma et trauma cérébral ou rachidien.

2. Utilisation selon la revendication 1 **caractérisée en ce que** les accident cérébro-vasculaire, ischémie, coma et trauma cérébral ou rachidien entraînent une dégénérescence des cellules du système nerveux central ou périphérique.

3. Utilisation selon les revendications 1 et 2 **caractérisée en ce que** les accidents cérébro-vasculaire, ischémie, coma et trauma cérébral ou rachidien constituent un état aigu dans lequel une cellule est soit entièrement privée d'oxygène suffisant et/ou des molécules qu'elle peut utiliser comme source d'énergie, soit est rendue incapable d'utiliser un tel oxygène ou une telle molécule par un dommage mécanique.

4. Utilisation selon les revendications 1 et 2, **caractérisée en ce que** les accident cérébro-vasculaire, ischémie, coma et trauma cérébral ou rachidien entraînent une inflammation, une élévation de la pression intracrânienne et une dégénérescence neuronale ou une apoptose entraînant une perte de la fonction cérébrale ou périphérique dans les sept jours d'établissement de la pathologie.

5. Utilisation selon l'une quelconque des revendications précédentes, **caractérisée en ce que** le traitement est prescrit à des fins de prophylaxie.

6. Utilisation selon les revendications 1 à 3, **caractérisée en ce que** le 7α-hydroxy-stéroïde est un estradiol (à savoir, un estra-1,3,5(10)-triène-3,7α,17β-triol) répondant à la formule générale Ia, 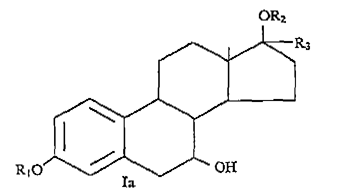 où OR₁ et OR₂ représentent chacun indépendamment un groupe hydroxy libre, un groupe éther ou un groupe hydroxy estérifié et R₃ est hydrogène, alkyle en C₁-C₆, alcényle en C₂-C₆ ou alcynyle en C₂-C₆ substitué ou non substitué,

7. Utilisation selon la revendication 6, **caractérisée en ce que** R₁ et R₂ sont choisis indépendamment parmi les groupes alkyle en C₁-C₆, substitués ou non substitués, chacun de ces substituants étant choisi dans le groupe constitué de OH, halogène (F, CI, Br, I), amino, alkylamino en C₁-C₆, dialkylamino en C₁-C₆, -COOH ou -COOR₅ où R₅ représente un groupe alkyle en C₁-C₆ qui peut être substitué ou non substitué par l'un des substituants auxquels on s'est référé ci-dessus; ou -OR₁ et OR₂ représentent chacun indépendamment un groupe hydroxy estérifié répondant à la formule R₆COO- dans laquelle R₆ peut être choisi parmi les groupes alkyle en C₁-C₆ substitués ou non substitués, chacun de tels substituants étant choisi dans le groupe constitué de OH, halogène (F, Cl, Br, I), amino, alkylamino en C₁-C₆, dialkylamino en C₁-C₆, -COOH ou - COOR₅ où R₅ représente un groupe alkyle en C₁-C₆; ou -OR₁ et OR₂ représentent chacun indépendamment un groupe hydroxy estérifié répondant à la formule -OP(OH)₃ ou un groupe sulfate,
ou un sel pharmaceutiquement acceptable d'un tel composé.

8. Utilisation selon les revendications 1 à 5, **caractérisée en ce que** le stéroïde est une 7α-hydroxy-DHEA ou un 3-acyloxy ester de ce composé.
